Europäisches Patentamt

European Patent Office

Office européen des brevets

(11) Veröffentlichungsnummer: **0 231 867**
**B1**

(12) **EUROPÄISCHE PATENTSCHRIFT**

(45) Veröffentlichungstag der Patentschrift:
12.09.90

(51) Int. Cl.⁵: **A61B 17/04,** D05B 27/18

(21) Anmeldenummer: 87101151.6

(22) Anmeldetag: 28.01.87

(54) **Chirurgische Nähmaschine.**

(30) Priorität: 30.01.86 DE 3602725
12.01.87 DE 3700639

(43) Veröffentlichungstag der Anmeldung:
12.08.87 Patentblatt 87/33

(45) Bekanntmachung des Hinweises auf die Patenterteilung:
12.09.90 Patentblatt 90/37

(84) Benannte Vertragsstaaten:
CH DE ES IT LI SE

(56) Entgegenhaltungen:
EP-A- 0 174 843
DE-A- 2 916 325
DE-A- 3 018 892
DE-C- 226 013
DE-C- 666 933
US-A- 2 988 028
US-A- 3 783 873
US-A- 4 123 982

(73) Patentinhaber: Pfaff Industriemaschinen GmbH,
Königstrasse 154, D-6750 Kaiserslautern(DE)

(72) Die Erfinder haben auf ihre Nennung verzichtet

(74) Vertreter: Klein, Friedrich, Königstrasse 154,
D-6750 Kaiserslautern(DE)

## Beschreibung

Die Erfindung betrifft eine chirurgische Nähmaschine mit einem Gehäuse sowie mit einer mit einem Nähfaden verbundenen und oszillierend angetriebenen Nadel, die mit einem Fadenfänger zusammenwirkt, und mit zwei Klemmteilen, zwischen denen die Ränder des zu vernähenden Gewebes einklemmbar sind und deren Klemmzone quer zum Nahtverlauf von der Nadel durchquert wird.

Eine chirurgische Nähmaschine dieser Art ist in DE-A 3 018 892 beschrieben. Sie weist eine oszillierende Nadel auf, durch deren Nadelöhr ein Nähfaden gezogen ist. Zum Vernähen von Geweberändern miteinander werden diese zwischen zwei Klemmteile eingeklemmt und so gehalten. Die beiden Klemmteile sind als gerade Schienen ausgebildet, die an dem einen Ende über ein Scharnier miteinander verbunden sind und die beim Halten der Geweberänder am anderen Ende mit Hilfe einer Arterienklemme o. dgl. zusammengehalten werden. Zwischen den beiden hochkant gegen die Körperfläche gerichteten Klemmteile, also in der Klemmzone, befinden sich dann die aufgerichteten und mit ihren Innenseiten aneinanderliegenden Geweberänder . Die Klemmteile weisen sich deckende und parallel zur Klemmzone verlaufende Längsschlitze auf, durch die die Nadel hindurchdringt, um das Gewebe zu durchstechen. Dabei durchdringt die oszillierend angetriebene Nadel die Klemmzone quer zum Nahtverlauf. Die Nadel wirkt mit einem Fadenfänger zusammen, wodurch die Naht erzeugt wird.

Beim Vernähen zweier Geweberänder müssen diese zunächst zwischen die Klemmteile eingeklemmt werden. Um die Naht zu erstellen, muß der die Nadel und den Fadenfänger aufweisende Teil der chirurgischen Nähmaschine auf die Klemmteile gesetzt werden. Zur Erstellung der Naht muß der die Nadel und den Fadenfänger aufweisende Teil der chirurgischen Nähmaschine nach jedem Durchstechen der Nadel durch die Geweberänder hindurch von Hand auf den Klemmteilen entlang der Klemmzone fortbewegt werden. Eine Überdecknaht entsteht hierdurch nicht, vielmehr handelt es sich nur um eine Folge von einzelnen Einstichen der Nadel durch die aufgerichteten Geweberänder und bei glattgestrichener Naht verläuft auf beiden Seiten der Schnittwunde je eine Steppstichreihe. Ferner ist keine Absicherung gegen Verletzung des Fadens möglich und die Naht öffnet sich unbeabsichtigt. Darüber hinaus können nur geradlinig verlaufende Nähte erstellt, d.h. nur geradlinig verlaufende Geweberänder miteinander vernäht werden. Die Länge einer zu erstellenden Naht ist durch die Klemmteile bzw. durch die Längsschlitze in den Klemmteilen bestimmt. Zum Erstellen von Nähten, die länger als die Klemmteile sind, müssen der Hauptteil der chirurgischen Nähmaschine von den Klemmteilen abgenommen, die Klemmteile gelöst und die noch zu vernähenden Geweberänder zwischen die Klemmteile eingeklemmt werden. Dieser Vorgang ist umständlich und verlängert die Operationszeit.

Durch die DE-A 2 916 325 ist eine Nähmaschine zum kontinuierlichen Bilden einer Sicherheitsnaht an zwei gegenüberliegenden Abschnitten zweier Werkstücklagen bekannt, bei denen es sich beispielsweise um Teile eines Taucheranzuges handeln kann. Die Nähmaschine weist zwei in einer gemeinsamen Ebene umlaufende Vorschubzylinder auf, zwischen denen ein zu einer Falte geformter Verbindungsabschnitt zweier Werkstücklagen hindurchgeführt und mit Hilfe einer parallel zur Umlaufebene der Vorschubzylinder oszillierenden Nadel genäht wird. Der in bezug auf das Maschinengehäuse äußere Vorschubzylinder ist am unteren Ende eines schwenkbar gelagerten rohrförmigen Trägers und der innere Vorschubzylinder am oberen Ende einer feststehenden Säule angeordnet. Aufgrund dieser Anordnung der Vorschubzylinder muß diejenige Werkstücklage, die sich von der Nahtlinie aus in Richtung des Maschinengehäuses erstreckt, senkrecht nach abwärts gebogen werden, da die den inneren Vorschubzylinder tragende Säule einer horizontalen Erstreckung dieser Werkstücklage im Wege steht. Aus diesem Grund ist die bekannte Nähmaschine für das Zusammennähen von Schnitträndern bei chirurgischen Operationen völlig ungeeignet.

Der Erfindung liegt die Aufgabe zugrunde, eine chirurgische Nähmaschine zu schaffen, mit der Geweberänder unabhängig von ihrem Verlauf und ihrer Länge fortlaufend und ohne zeitliche Unterbrechungen miteinander vernäht werden können. Die Aufgabe wird durch die kennzeichnenden Merkmale des Anspruches 1 gelöst.

Die erfindungsgemäße chirurgische Nähmaschine weist zumindest einen Klemmteil auf, der als drehbarer zylindrischer Körper ausgebildet ist. Der andere Klemmteil kann unbeweglich sein. Da die beiden Klemmteile am Gehäuse befestigt sind und ihre dem zu vernähenden Gewebe zugewandten Stirnseiten freie Gleitfläche bilden, können die zu vernähenden Geweberänder zwischen den beiden Klemmteilen hochstehend und aneinander anliegend partiell eingeklemmt werden, ohne daß dabei die links und rechts der Geweberänder im wesentlichen quer verlaufenden Körperpartien im Wege wären.

Beim Vorwärtsbewegen der chirurgischen Nähmaschine längs der Naht dreht sich der zylindrische Körper, wobei seine Umfangsfläche auf der Außenfläche des einen Geweberandes abrollt, während die Außenfläche des anderen Geweberandes an dem gegenüberliegenden ggf. unbeweglichen Klemmteil vorbeibewegt wird. Die Oberkante der hochstehend eingeklemmten Geweberänder liegt zwischen den diese überfahrenden Klemmteilen frei, so daß beliebige Überdecknähte gebildet werden können, die sich in der Chirurgie als besonders vorteilhaft erwiesen haben, weil sie haltbar und trotzdem leicht lösbar sind.

Bei der erfindungsgemäßen chirurgischen Nähmaschine brauchen die zu vernähenden Geweberänder vor dem Vernähen über ihre gesamte Länge nicht zwischen die Klemmteile eingeklemmt zu werden. Es wird immer nur derjenige Teil der Geweberänder eingeklemmt, der gerade vernäht wird. Beim Vorwärtsbewegen der chirurgischen Nähmaschine drückt der Umfang des sich drehenden zylindrischen Körpers gegen die Außenfläche des einen hochstehenden Geweberandes, dessen Innenfläche somit gegen die Innenfläche des anderen außen abgestützten Geweberandes gedrückt wird. Im Bereich des kürzesten Abstandes zwischen dem Umfang des zylindrischen Körpers und dem anderen Klemmteil werden die beiden Geweberänder eingeklemmt gehalten. Das Einklemmen erfolgt also beim Fortbewegen der chirurgischen Nähmaschine kontinuierlich automatisch, so daß Nähte beiliebiger Länge unterbrechungslos angelegt werden können. Auch ist es möglich, Geweberänder zu vernähen, deren Verlauf beliebig ist. Die zu vernähenden Geweberänder können krummlinig z. B. S-förmig verlaufen. Infolge des mindestens einen drehbaren zylindrischen Körpers kann durch entsprechendes Fortbewegen der chirurgischen Nähmaschine dem Verlauf der Geweberänder mühelos gefolgt werden.

Die mit Hilfe der chirurgischen Nähmaschine erstellte Naht wird ohne Unterbrechung, d. h. ohne Umsetzen der chirurgischen Nähmaschine, in einem Nähvorgang erstellt. Die Geweberänder werden sicher und zeitsparend maschinell mit Überdecknaht vernäht, wodurch sich die Operationszeit verkürzt. Der Antrieb des drehbaren zylindrischen Körpers erfolgt entweder durch das Vorschieben der Nähmaschine, wobei sich der Umfang des zylindrischen Körpers auf der Außenfläche eines Geweberandes abrollt und der zylindrische Körper drehend bewegt wird. Eine andere Möglichkeit besteht darin, einen gesonderten Antrieb für den mindestens einen zylindrischen Körper vorzusehen.

Gemäß einer vorteilhaften Ausgestaltung der Erfindung ist vorgesehen, daß die obere Stirnseite des zylindrischen Körpers über eine Drehachse mit einer Verstellvorrichtung zum Ausrücken oder Einrücken des zylindrischen Körpers in bezug auf den zweiten Klemmteil hängend verbunden ist, und daß die untere Stirnseite des zylindrischen Körpers eine im wesentlichen freie Gleitfläche bildet. Mit Hilfe der Verstellvorrichtung kann der zylindrische Körper von dem zweiten Klemmteil abgerückt werden, um zu Beginn des Nähvorganges die hochstehenden Geweberänder zwischen die Klemmteile bringen zu können. Anschließend wird der zylindrische Körper mit Hilfe der Verstellvorrichtung zu dem zweiten Klemmteil hinbewegt und die Geweberänder eingeklemmt. Die untere Stirnseite des zylindrischen Körpers kann glattflächig sein. Diese untere Stirnseite berührt beim Nähvorgang die Haut des Patienten, die an denjenigen Geweberand angrenzt, auf dessen Außenfläche der Umfang des drehbaren zylindrischen Körpers abrollt. Durch die Verstellvorrichtung sowie die von Maschinenbauteilen freie untere Stirnseite des zylindrischen Körpers wird eine einfache Handhabung der chirurgischen Nähmaschine sowohl beim "Einspannen" der Geweberänder zu Beginn des Nähvorganges als auch beim Vorwärtsschieben der Nähmaschine erreicht.

Zweckmäßigerweise ist vorgesehen, daß die Verstellvorrichtung eine federelastische, einstellbare Einrichtung zum Ändern der Klemmkraft des zylindrischen Körpers aufweist. Hierdurch kann die Andrückkraft des zylindrischen Körpers auf das zweite Klemmteil eingestellt werden. Die Andrückkraft kann somit unterschiedlich dicken Geweberändern entsprechend angepaßt werden.

Eine vorteilhafte Weiterbildung der Erfindung ist dadurch gekennzeichnet, daß der zylindrische Körper an der Einlaufseite des Gewebes in die Klemmzone abgesenkt ist und daß der Umfang des zylindrischen Körpers als Mitnehmerring profiliert ist. Durch Schrägstellung der Drehachse ist der in Bewegungsrichtung der chirurgischen Nähmaschine weisende Teil des zylindrischen Körpers nach unten geneigt und der diesem Teil diametral gegenüberliegende Teil des zylindrischen Körpers entsprechend angehoben. Die Position des zylindrischen Körpers in bezug auf die Nadel ist dabei derart, daß die Nadel sich nach dem Durchdringen der Geweberänder über die obere Stirnfläche des zylindrischen Körpers frei hinwegbewegen kann. Der Umfang des zylindrischen Körpers ist so profiliert, daß das Mitnehmen des Geweberandes, auf dessen Außenfläche der Umfang abrollt, verbessert wird. Auch können hervorstehende und zurückgehende spitze Stifte oder Saugnäpfe auf dem Umfang angeordnet sein. Beim Vorwärtsbewegen der chirurgischen Nähmaschine wird der Geweberand von dem als Mitnehmerring ausgebildeten Umfang des zylindrischen Körpers in dessen in Fortbewegungsrichtung der chirurgischen Nähmaschine weisenden Teil "gefaßt" und zusammen mit dem am zweiten Klemmteil anliegenden hochstehenden Geweberand angehoben, so daß die Geweberänder im Einstichbereich der Nadel, d. h. in der Klemmzone der Klemmteile, über den Klemmteilen überstehen. Durch die Schrägstellung des zylindrischen Körpers sowie der besonderen Ausgestaltung seines Umfanges wird demnach ein sicheres Einlaufen des Gewebes in die Klemmzone erreicht.

In vorteilhafter Weiterbildung der Erfindung ist vorgesehen, daß der zylindrische Körper eine kreisförmige Scheibe ist. Dadurch kann die chirurgische Nähmaschine kompakt und in ihren Abmessungen klein gemacht werden. Statt einer kreisförmigen Scheibe kann auch eine Walze, eine Rolle oder ein Band verwendet werden.

Bei einer weiteren Ausgestaltung der Erfindung ist an der oberen Stirnseite des zylindrischen Körpers eine Nadelführungsfläche und/oder eine unbewegliche Nadelführung angeordnet. Beim Nähvorgang bewegt sich die Nadel nach dem Durchstechen der Geweberänder über die obere Stirnseite des zylindrischen Körpers hinweg. Um die Nadel gegen quer zu ihrer Oszillationsrichtung verlaufende Auslenkungen zu sichern, kann die obere Stirnseite des zylindrischen Körpers als Nadelführungsfläche zum Führen der Nadel ausgebildet sein. Eine andere Möglichkeit besteht darin, daß über der oberen Stirnseite eine Nadelführung angeordnet ist, die ein Auslenken der Nadel verhindert. Die Nadelführung ist unbeweglich und derart angeordnet, daß sich der zylindrische Körper unter ihr dreht. Durch die beiden Maßnahmen wird erreicht, daß die Bewegung der Nadel stabilisiert wird. Das wiederum hat zur Folge, daß die Einstichlöcher in den Geweberändern nicht aufgeweitet werden, da ein Abweichen der Oszillationsrichtung der Nadel nach dem Durchdringen der Geweberänder verhindert wird.

Bei einer weiteren vorteilhaften Ausgestaltung der Erfindung ist vorgesehen, daß an der oberen Stirnseite des zylindrischen Körpers im Bereich der Klemmzone eine unbewegliche Stützvorrichtung für Gewebeüberstand angeordnet ist. Durch die Stützvorrichtung wird verhindert, daß der Gewebeüberstand (derjenige Teil der Geweberänder, der über den Klemmteilen übersteht) beim Durchdringen der Nadel niedergedrückt wird. Somit wird erreicht, daß die Geweberänder in gleicher Höhe von der Nadel durchstochen werden und eine gleichmäßige einwandfreie, sichere Naht entsteht. Die Stützvorrichtung kann an beiden Klemmteilen angeordnet sein. Durch eine derartige Stützvorrichtung wird verhindert, daß sich der Gewebeüberstand sowohl beim Einstechen der Nadel als auch beim Herausbewegen der Nadel mit dieser bewegt.

Bei einer anderen vorteilhaften Ausgestaltung der Erfindung ist vorgesehen, daß an der Auslaufseite des Gewebes aus der Klemmzone ein Glattstreicher für die Naht angeordnet ist. Der Glattstreicher ist in bezug auf die Bewegung der chirurgischen Nähmaschine hinter der Nadel angeordnet und drückt den zuletzt erstellten Nahtabschnitt flach. Durch den Glattstreicher werden die hochstehenden und aneinanderliegenden miteinander vernähten Geweberänder heruntergedrückt, so daß sich ihre Kanten stumpf aneinanderstoßend gegenüberliegen. In diesem Zustand können die Geweberänder dann zusammenwachsen.

Vorteilhafterweise weist der Glattstreicher einen elliptisch bewegbaren Andrückschuh auf. Dieser ist beispielsweise über einen Exzenter angetrieben und in seiner Schrittlänger einstellbar. Der Andrückschuh gleitet über die Naht und glättet diese. Er bewegt sich auf einer elliptischen Bahn, derart, daß er die Naht bei seiner Bewegung in Vorwärtsrichtung der Nähmaschine nicht berührt, während er die Naht bei seiner Bewegung entgegengesetzt zur Bewegungsrichtung der chirurgischen Nähmaschine niederdrückt. Hierdurch wird erreicht, daß die Geweberänder immer nur dann flachgedrückt werden, wenn sich der Andrückschuh mit der Naht mitbewegt. Dies führt zu einem besonders schonenden Flachdrücken des jeweils zuletzt genähten Nahtteiles. Beim Glattstreichen der Naht bewirkt der Glattstreicher bzw. der Andrückschuh auch einen absatzweisen Vorschub der chirurgischen Nähmaschine.

Bei einer weiteren vorteilhaften Ausgestaltung der Erfindung ist der Fadenfänger als gegabelter Arm ausgebildet, der sich axial und auf einer Teilkreisbahn bewegt und mit der Nadel synchron so angetrieben wird, daß der Fadenfänger den Nähfaden nach einer Durchdringung der Nadel durch das Gewebe von dieser aufnimmt, ihn über die Klemmzone legt und vor dem nächsten Durchdringen der Nadel durch das Gewebe in Richtung der Einlaufseite des Gewebes in die Klemmzone vor die Nadel legt. Durch dieses Zusammenwirken von Nadel und Fadenfänger wird eine Einfadennaht erzeugt, bei der die Schnittstelle, d. h. die aneinanderstoßenden Geweberänderkanten, von einer Schlinge überdeckt ist. Nachdem die Nadel durch die überstehenden Geweberänder hindurchgedrungen ist und dabei den Nähfaden durchgezogen hat, kehrt sich die Bewegungsrichtung der Nadel um, so daß sich die Nadel zurückbewegt. Aufgrund der Reibung des Nähfadens im Gewebe wirft sich dieser auf, so daß eine Schlaufe entsteht. Beim Zurückbewegen der Nadel bewegt sich der Fadenfänger auf seiner axialen Bahn in die Schlaufe hinein und nimmt so den Faden von der Nadel auf. Während sich die Nadel weiter aus dem Gewebe herausbewegt, beschreibt der Fadenfänger eine Teilkreisbahn, wobei er die Schlaufe über die Geweberänder auf die Einstichseite des Gewebes führt. Während sich der Fadenfänger auf seiner Teilkreisbahn bewegt, wird die chirurgische Nähmaschine relativ zur Naht bewegt, so daß die Nadel nicht an der gleichen Stelle in das Gewebe hineinsticht. Nachdem der Fadenfänger die Fadenschlaufe über die Geweberänder und in Richtung auf die Einlaufseite des Gewebes in die Klemmzone vor die Nadel gelegt hat, bewegt sich der Fadenfänger in axialer Richtung zurück. Die sich dabei auf die Geweberänder zu bewegende Nadel dringt durch die Fadenschlaufe hindurch und in das Gewebe ein. So entsteht eine Einfaden-Blindstich-Kettenstich-Naht, die die Schnittstelle, d. h. die Geweberänder, überdeckt. Es ist jedoch auch möglich, die Blindstich-Kettenstich-Naht mit zwei Fäden zu erstellen. Dabei würde dann der Fadenfänger entsprechend mit einem zweiten Nähfaden zusammenwirken. Aufgrund der Blindstich-Kettenstich-Naht ist es möglich, daß die während des Nähvorganges aneinanderliegenden und hochstehenden Geweberänder nach dem Zusammennähen durch den Glattstreicher flachgedrückt werden können, so daß die Gewebekanten sich gegenüberliegen und stumpf aneinander anstoßen In diesem Zustand können die Gewebekanten dann zusammenwachsen. Darüber hinaus hat die Einfaden-Blindstich-Kettenstich-Naht die Eigenschaft, daß die gesamte Fadenmenge herausgezogen werden kann, wenn die Naht an der richtigen Stelle getrennt wird. Damit ergibt sich ein besonders einfaches "Fadenziehen". Wird die Naht dagegen nicht an dieser sondern an einer anderen Stelle getrennt, so ist sie automatisch gegen Auftrennen gesichert. Ebenfalls kann beim Vernähen der Faden nach Beendigung eines oder mehrerer Stiche an einer vorbestimmten Stelle abgeschnitten und dadurch automatisch verknotet werden.

Eine weitere vorteilhafte Ausgestaltung der Erfindung ist dadurch gekennzeichnet, daß in dem Gehäuse der chirurgischen Nähmaschine Antriebs- und Übertragungselemente für den zylindrischen Körper, für einen Nadelhalter und für den Fadenfänger angeordnet sind, daß das Gehäuse mit einem Griffstück versehen ist und daß an der Kopfseite des Gehäuses der zylindrische Körper, der Nadelhalter und der Fadenfänger mittels Schnellverschlüssen jeweils lösbar angeordnet sind. Die zu sterilisierenden Elemente am Kopfstück des Gehäuses der chirurgischen Nähmaschine können schnell und einfach mit Hilfe der Schnellverschlüsse lösbar angebracht werden. Dadurch ist ein problemloses und schnelles Auswechseln der Teile möglich. Hierdurch wird die Sterilisation der chirurgischen Nähmaschine erleichtert.

Der Antrieb der chirurgischen Nähmaschine erfolgt über einen üblichen Elektromotor mit Nadelhochstellung, so daß sowohl Einzelstiche als auch Stichfolgen genäht werden können. Außerdem erlaubt dieser Motor den Anschluß eines Fadenabschneiders zum Trennen der Kette. Dies kann auch von Hand mit einer Schere erfolgen.

Um zu vermeiden, daß die zwischen den Klemmteilen eingespannten und durch die sich trichterförmig verengenden Stützfinger seitlich gehaltenen Geweberänder gequetscht werden, ist bei einer alternativen Ausgestaltung der Erfindung im Stichbildebereich der Nadel wenigstens eine während eines jeden Stichbildevorganges wirksame Saugluft-Haltevorrichtung für die Geweberänder vorgesehen. Durch die mit Saugluft arbeitende Haltevorrichtung werden die Schnittkanten der miteinander zu verbindenden Geweberänder während jedes Stichbildevorganges so festgehalten, daß die beim Ein- und Durchstechen sowie beim Zurückziehen der Nadel auf die Geweberänder einwirkenden seitlich gerichteten Kräfte von der Haltevorrichtung aufgefangen werden, so daß die Klemmkraft der die Geweberänder haltenden Klemmteile verringert werden kann und die Stützfinger vollständig entfallen können. Auf diese Weise werden Quetschungen des Gewebes zuverlässig vermieden. Eine weitere Verringerung der Klemm- bzw. Andruckkraft der Klemmteile läßt sich dann erzielen, wenn das bzw. die als Räder ausgebildeten Klemmteile an der Umfangsseite mit radial abstehenden Nadeln versehen sind, da in diesem Fall eine formschlüssige Mitnahmeverbindung zwischen den Klemmteilen und den Geweberändern besteht.

Gemäß einer weiteren Ausgestaltung der Erfindung ist die Haltevorrichtung durch einen Antriebsmechanismus in vertikaler Ebene auf- und abbewegbar. Durch diese Maßnahme besteht die Möglichkeit, jeweils die im Stichbildebereich liegenden Abschnitte der Geweberänder genau auf die für eine einwandfreie Stichbildung erforderliche Höhe bezüglich der Bewegungsbahn der Nadel anzuheben. Durch eine entsprechende zeitliche Überlagerung der Auf- und Abbewegung der Haltevorrichtung mit der Steuerung der nur während der einzelnen Stichbildevorgänge wirksamen Saugluft für die Haltevorrichtung läßt sich erreichen, daß die Geweberänder und die Haltevorrichtung in der Zeit zwischen je zwei Stichbildevorgängen, d. h. in der Zeit in welcher sich die Nadel außerhalb des Gewebes befindet, voneinander getrennt sind. Auf diese Weise läßt sich nach jedem Stichbildevorgang die Vorschubbewegung der Nähmaschine gegenüber dem ortsfesten Gewebe störungsfrei durchführen.

Nach einer weitergehenden Ausgestaltung der Erfindung ist der Antriebsmechanismus der Haltevorrichtung mit dem Triebwerk der Nähmaschine antriebsmäßig verbunden und die Hubhöhe der Haltevorrichtung einstellbar. Durch die antriebsmäßige Verbindung des Antriebsmechanismus mit dem Nähmaschinentriebwerk und zwar vorzugsweise mit dem Nadelstangenantrieb, wird nicht nur die Antriebsbewegung für die Haltevorrichtung von einem ohnehin vorhandenen Getriebeteil der Nähmaschine abgenommen sondern zugleich auch eine Synchronisierung der Bewegung der Haltevorrichtung mit der Nadelbewegung erzielt. Durch die Einstellung der Hubhöhe der Haltevorrichtung läßt sich der Abstand der Einstiche der Nadel zu den Schnittkanten der Geweberänder und damit der Nahtabstand verändern.

Durch die weitere Ausbildung der Erfindung, wonach die Haltevorrichtung ein im Takt der Nadelbewegung steuerbares Ventil für die Zuleitung und Absperrung der Saugluft aufweist, wird auch die Steuerung der Saugluft mit der Nadelbewegung synchronisiert.

Aufgrund der weiteren Ausbildung der Erfindung, wonach die Haltevorrichtung einen entgegen der Vorschubrichtung der Nähmaschine flach auslaufenden Saugfuß mit einer an dessen Unterseite ausgebildeten Ansaugöffnung aufweist, kann die die Schnittkanten der Geweberänder einfassende Überdecknaht teilweise über den Saugfuß hinweg gebildet werden, so daß dieser direkt im Stichbildebereich angeordnet sein kann. Die Stichbildung geschieht in diesem Fall in der Weise, daß zunächst die Nadel unterhalb des Saugfußes in die angehobenen Geweberänder einsticht und durch sie hindurchdringt und dabei den Nähfaden mit durchzieht. Während des Zurückziehens der Nadel bildet sich aufgrund der Reibung des Nähfadens im Gewebe eine Fadenschlaufe aus, die der Fadenfänger aufnimmt, über die Schnittkanten und das Saugrohr hinweghebt und auf der gegenüberliegenden Seite vor die wiedereinstechende Nadel legt. Vor dem Wiedereinstechen wird die Nähmaschine um das Maß der Stichlänge in Vorschubrichtung relativ zum Gewebe bewegt, wodurch die über dem Saugrohr gebildete Fadenschlaufe abgezogen wird.

Nachfolgend werden unter Bezugnahme auf die Zeichnung zwei Ausführungsbeispiele der Erfindung näher erläutert.

Es zeigen:

Fig. 1 eine Vorderansicht der chirurgischen Nähmaschine,
Fig. 2 eine Seitenansicht der chirurgischen Nähmaschine nach Fig. 1, bei geöffnetem Gehäusedeckel,
Fig. 3 einen Schnitt entlang der Linie III-III der Fig. 1,
Fig. 4 einen Schnitt entlang der Linie IV-IV der Fig. 1,
Fig. 5 einen Schnitt entlang der Linie V-V der Fig. 1,
Fig. 6 die zwischen den Klemmteilen eingeklemmten hochstehenden Geweberänder, durch die gerade die Nadel hindurchsticht.
Fig. 7 eine Draufsicht auf die Klemmteile mit zwischen ihnen eingeklemmten hochstehenden Geweberändern,
Fig. 8 die Draufsicht auf zwei drehbare zylindrische Körper, die als Klemmteile fungieren und mit einer Stützvorrichtung versehen sind,
Fig. 9 die Vorderansicht der beiden drehbaren zylindrischen Körper nach Fig. 8,
Fig. 10 einen Querschnitt durch zwei als Klemmteile fungierende drehbare zylindrische Körper,
Fig. 11 einen Schnitt entlang der Linie XI-XI der Fig. 10 und

Fig. 12 eine schematische Darstellung der Fadennaht.

Fig. 13 eine teilweise geschnittene Ansicht des zweiten Ausführungsbeispiels einer chirurgischen Nähmaschine mit einer Haltevorrichtung für die Geweberänder,

Fig. 14 eine Ansicht eines Teiles des Antriebsmechanismus für den Fadenfänger,

Fig. 15 eine Vorderansicht der chirurgischen Nähmaschine,

Fig. 16 eine Ansicht des Antriebsmechanismus für die Haltevorrichtung,

Fig. 17 eine Vorderansicht eines Teils der Transporträder und der Nadel während des Nähens.

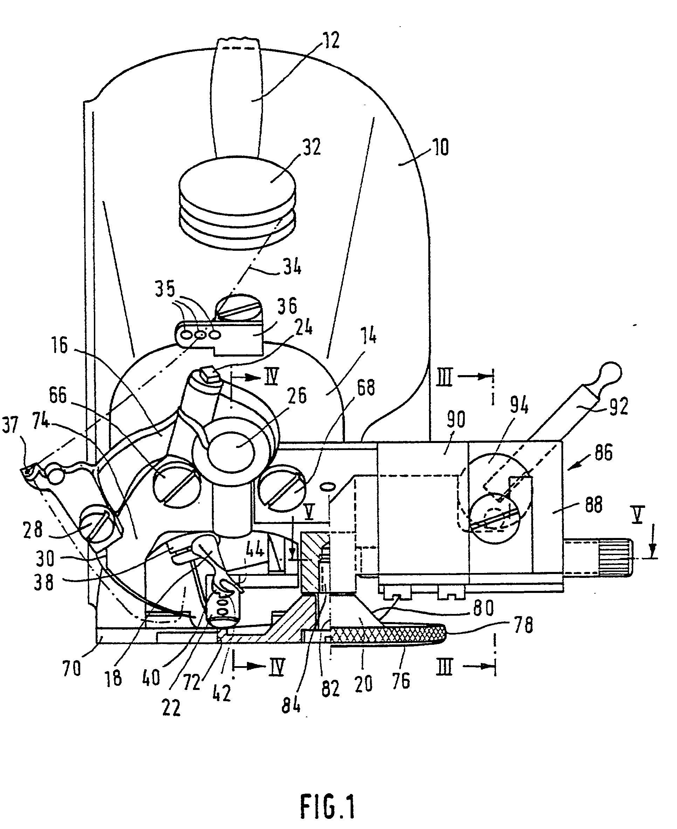

In Fig. 1 ist die Vorderansicht der chirurgischen Nähmaschine dargestellt. Sie weist ein Gehäuse 10 auf, an dessen Oberseite ein Handgriff 12 angeordnet ist. An der linken Seite des Gehäuses 10 befindet sich ein (nicht dargestellter) abnehmbarer Gehäusedeckel. An der Kopfseite (14) des Gehäuses (10) der chirurgischen Nähmaschine sind ein Nadelhalter (16), ein Fadenfänger (18) und ein kreiszylindrischer scheibenförmiger Körper (20) angeordnet. Desweiteren befindet sich an der Kopfseite (14) des Gehäuses (10) ein glattstreicher (22). Der Nadelhalter (16) weist eine Schelle auf, die mit Hilfe einer Schraube (24) an einer oszillierenden Antriebswelle (26), die aus dem Kopfstück (14) herausragt, befestigt ist. Am Nadelhalter (16) wird mit Hilfe einer Schraube (28) eine Nähnadel (30) befestigt. Bei Oszillation der Welle (26) beschreibt die Nadelspitze, in der sich das Nadelöhr (32a) befindet, eine Kreisbahn. Bei der Nähnadel (30) handelt es sich um eine Rundnadel.

Auf der Oberseite des Gehäuses (10) ist vor dem Handgriff (12) eine Rolle (32) drehbar angeordnet, auf der der Nähfaden (34) aufgewickelt ist. Der Nähfaden (34) verläuft von der Rolle (32) über einen Fadenhalter (36) durch ein Durchgangsloch am Nadelhalter (16) zur Spitze der Nähnadel (30), wo er durch das Nadelöhr (32a) gezogen ist. Der Fadenhalter (36) ist als ein mit mindestens einem Durchgangsloch versehenes Winkelstück ausgebildet, das an der am Kopfstück (14) angrenzenden Kante der Oberseite des Gehäuses (10) festgeschraubt ist. Der eine Schenkel des Winkelstückes ragt über das Kopfstück (14) hinaus und weist nach oben. Das Durchgangsloch (37) am Nadelhalter (16) ist an demjenigen Ende des Nadelhalters (16) angeordnet, an dem die Nähnadel (30) befestigt ist. Der Fadenfänger (18) ist im Inneren des Gehäuses an einer Klemmvorrichtung (38) befestigt und ragt aus der Kopfseite (14) der chirurgischen Nähmaschine unterhalb der Welle (26) heraus. Er ist als abgewinkelter Arm ausgebildet, dessen vorderes freies Ende gegabelt ist. Die eine Zinke (40) des gegabelten Endes des Fadenfängers (18) ist gebogen und weist einen Haken (42) auf, während die zweite Zinke (44) nahezu gerade verläuft. Der Bewegungsablauf und die Funktionsweise des Fadenfängers sowie der Nähnadel werden später erläutert.

In dem Gehäuse (10) der chirurgischen Nähmaschine befinden sich die Antriebs- und Übertragungselemente für den Nadelhalter (16), den Fadenfänger (18) und den Glattstreicher (22). Die Welle (26) stellt das Übertragungselement für die Schwenkbewegung des Nadelhalters (16) dar und verläuft durch eine Bohrung (46) im Kopfstück (14) des Gehäuses (10). In die Bohrung (46) ist eine Hülse (48) eingesetzt, in der die Welle (26) gelagert ist. Im Inneren des Gehäuses (10) befindet sich ein einstückig mit dem Gehäuse (10) geformter Vorsprung (50), der eine Durchgangsbohrung (52) aufweist, in die eine Hülse (54) eingepaßt ist. Die Welle (26) ist in der Hülse (54) gelagert und führt von dort aus weiter zu einem (nicht dargestellten) Antrieb, durch den die Welle (26) hin- und hergedreht wird. Diese Drehbewegung der Welle (20) führt dazu, daß die von dem Nadelhalter (16) gehaltene Nähnadel (30) oszilliert.

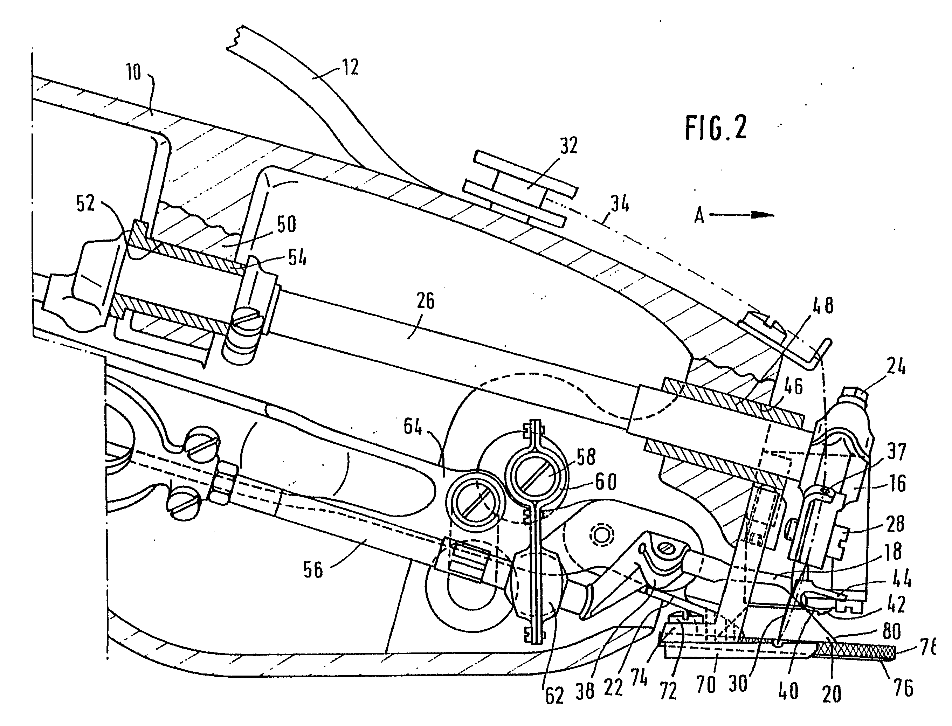

Wie bereits zuvor erwähnt, ist der Fadenfänger (18) an einer Klemmvorrichtung (38) im Inneren des Gehäuses (10) befestigt. Die Klemmvorrichtung (38) ist mit einem Hebel (56) verbunden, der wiederum über ein Kreuzgelenk (nicht dargestellt) mit einer Antriebswelle verbunden ist. In dem Gehäuse (10) befindet sich ein an einem Bolzen (58) drehbar gelagerter Hebel (60), an dessen freiem Ende ein Kugellager (62) angeordnet ist, das den Hebel (56) lagert. Die Drehbewegung der Antriebswelle wird durch das Kreuzgelenk in eine Hin- und Herbewegung des Hebels (56) überführt, wobei dieser Bewegung des Hebels (56) eine Drehbewegung um seine Längsachse überlagert ist. Während der Bewegung des Hebels (56) dreht sich dieser im Kugellager (62), wobei der Hebel (60) um den Bolzen (58) geschwenkt wird. In dem Gehäuse (10) der chirurgischen Nähmaschine befindet sich ein zweiter Hebel (64), an dessen vorderem Ende der Glattstreicher (22) befestigt ist und der aus der Kopfseite (14) des Gehäuses (10) herausragt. Der Hebel (64) ist mit einer Antriebswelle verbunden, die ihn derart bewegt, daß der Glattstreicher (22) bei Betrachtung der chirurgischen Nähmaschine von der Seite (Fig. 2) eine elliptische Bahnkurve beschreibt. Die Klemmteile der chirurgischen Nähmaschine, zwischen denen die hochstehenden Geweberänder beim Vernähen kurzzeitig eingeklemmt werden, sind mit jeweils einer Schraube (66) und (68) am Kopfstück (14) des Gehäuses (10) befestigt. Dabei ist das erste Klemmteil als unbewegliche Platte (70) ausgebildet, an deren einer gerader Längskante (72) die Außenfläche eines Geweberandes vorbeigleitet. Die Platte (70) ist mittels einer Schraube (72) an einem Schenkel eines Winkelstückes (74) befestigt, dessen anderer Schenkel mit Hilfe der Schraube (66) am Kopfstück (14) befestigt ist. Die Platte (70) stellt demnach den unbeweglichen der beiden Klemmteile dar.

Der zweite Klemmteil ist als drehbarer zylindrischer Körper (20) ausgebildet, der bei deisem Ausführungsbeispiel der chirurgischen Nähmaschine eine Scheibe (76) ist. Die Scheibe (76) ist in bezug auf die feststehende Platte (70) derart angeordnet, daß ihre zylindrische Umfangsfläche (78) der Längskante der Platte (70), an der die Außenfläche des einen Geweberandes vorbeigleitet, zugewandt ist. Die Unterseite der Scheibe (76), die beim Vernähen der Geweberänder auf der Haut aufliegt, ist glattflächig. Die obere Stirnseite der Scheibe (76) weist in der Mitte eine kegelstumpfförmige Erhebung (80) auf, die sich nach oben verjüngt. Die obere Stirnseite der Erhebung (80) ist mit einer Drehachse (84) derart verbunden, daß diese mit der Mittelachse (82) der Scheibe (76) koaxial ist. Die Drehachse (84) ist drehbar und lösbar in ein Sackloch einer Verstellvorrichtung (86) eingesetzt, die mittels der Schraube (68) an der Stirnseite (14) des Gehäuses (10) befestigt ist. Die Verstellvorrichtung (86) besteht aus zwei gegeneinander verschiebbar gehaltenen Teilen (88) und (90), wobei das Teil (88) mit der Scheibe (76) und das Teil (90) mit der Schraube (68) an der Kopfseite (14) des Gehäuses (10) befestigt ist.

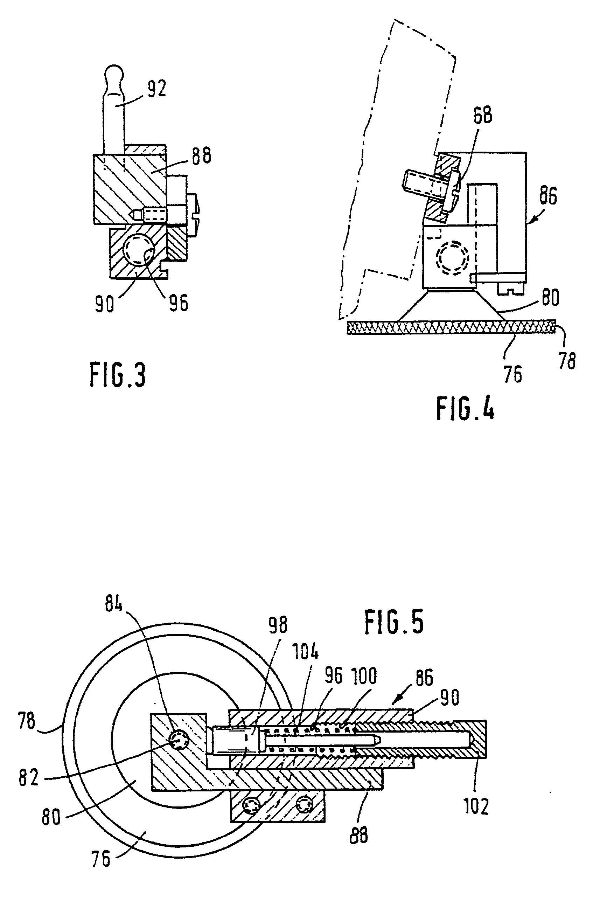

Mit Hilfe eines Hebels (92), der schwenkbar an dem Teil (90) der Verstellvorrichtung (86) angeordnet ist, kann das Teil (88) gegen das Teil (90) verschoben werden, so daß die Scheibe (76) auf die Platte (70) zubewegt oder von dieser wegbewegt wird. Der Hebel (92) ist mit einer Exzenterscheibe (94) verbunden, die am Teil (90) drehbar gelagert ist. Der Umfang der Exzenterscheibe (94) berührt das Teil (88), so daß beim Schwenken des Hebels (92) das Teil (88) in bezug auf das Teil (90) verschoben wird. Durch das Teil (90) verläuft radial zur Drehachse (84) der Scheibe (76) eine Durchgangsbohrung (96), in die ein Kolben (98) eingesetzt ist (Fig. 5). Das der Rotationswelle (84) abgewandte Ende der Durchgangsbohrung (96) ist mit einem Innengewinde (100) versehen, das mit dem Außengewinde einer Schraube (102) im Eingriff steht. Zwischen dem Kolben (98) und der Schraube (102) befindet sich eine Schraubenfeder (104), durch die das Teil (88) der Verstellvorrichtung (86) über den Kolben (98) in Richtung auf die Platte (70) vorgespannt wird. Mit Hilfe der Schraube (102) kann die Kraft, die die Schraubenfeder (104) auf den Kolben (98) und damit auf das Teil (88) ausübt, eingestellt werden. Beim Schwenken des Hebels (92) in Richtung auf das Gehäuse (10) wird die Scheibe (76) gegen die Kraft der Schraubenfeder (104) von der Platte (70) wegbewegt.

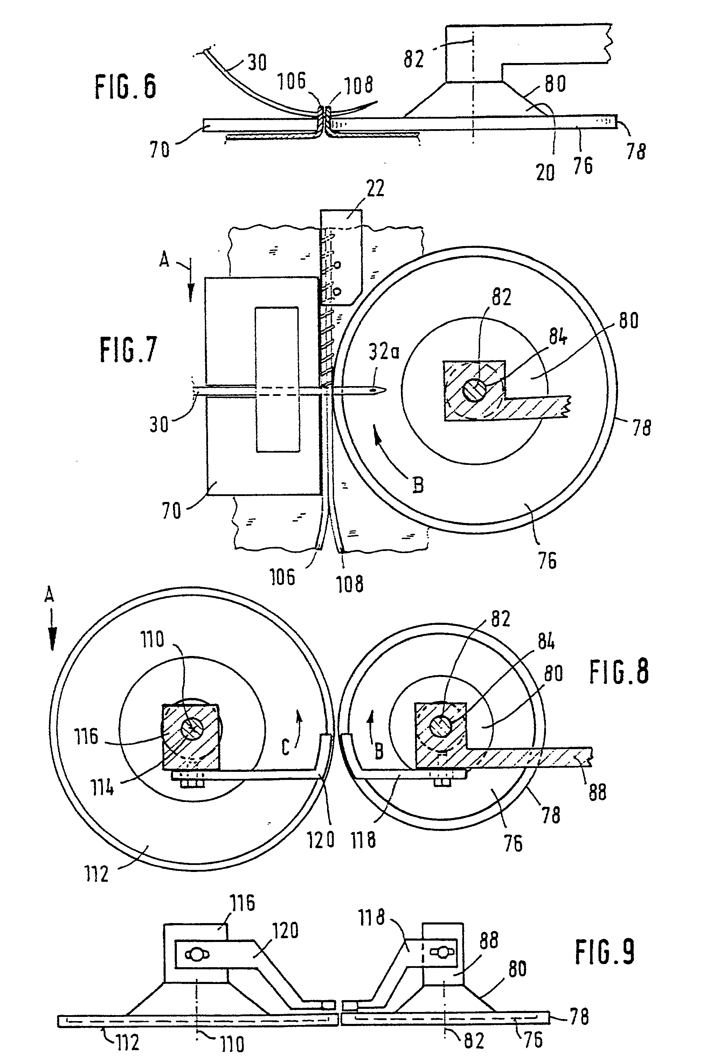

Beim Vernähen werden die Geweberänder (106) und (108) gemäß Fig. 6 aufrechtstehend und aneinanderliegend zwischen der Platte (70) und der Scheibe (76) partiell eingeklemmt. Dabei gleitet die Außenfläche des Geweberandes (106) an der der Scheibe (76) zugewandten Längskante der Platte (70) entlang, während der Umfang (78) der Scheibe (76) auf der Außenfläche des Geweberandes (108) abrollt. Der über die Längskante der Platte (70) und die Scheibe (76) überstehende freie Bereich der Geweberänder wird von der Nadel (30) durchstochen und mit dem Nähfaden vernäht. Fig. 7 zeigt eine Draufsicht auf die beiden zu vernähenden Geweberänder (106) und (108) und verdeutlicht, wie die beiden hochstehenden Geweberänder beim Bewegen der chirurgischen Nähmaschine in Richtung des Pfeiles A, in die Klemmzone zwischen dem feststehenden Fuß mit der Platte (70) und der Scheibe (76) "einlaufen". Bei Bewegung der Nähmaschine in Richtung des Pfeiles A dreht sich die Scheibe (76) in Richtung des Pfeiles B. Die Umfangsfläche (78) der Scheibe (76) ist, wie in den Fign. 1, 2 und 4 dargestellt, als grobes Kreuzraster profiliert. Die Umfangsfläche (78) "verhakt" sich mit der Außenfläche des Geweberandes (108) und zieht diesen zusammen mit dem Geweberand (106) in die Klemmzone, d.h. in den Bereich, in dem der Abstand zwischen der Platte (70) und der Scheibe (76) sehr klein ist. Aufgrund der in Fig. 2 dargestellten Schrägstellung der Scheibe (76) in bezug auf den feststehenden Fuß der chirurgischen Nähmaschine wird der Geweberand (108) zusammen mit dem Geweberand (106) beim Einziehen in die Klemmzone angehoben, so daß die Gewebränder ausreichend weit über die Platte (70) und die Scheibe (76) überstehen. Durch diesen Gewebeüberstand sticht die Nadel (30) beim Vernähen der beiden Geweberänder hindurch.

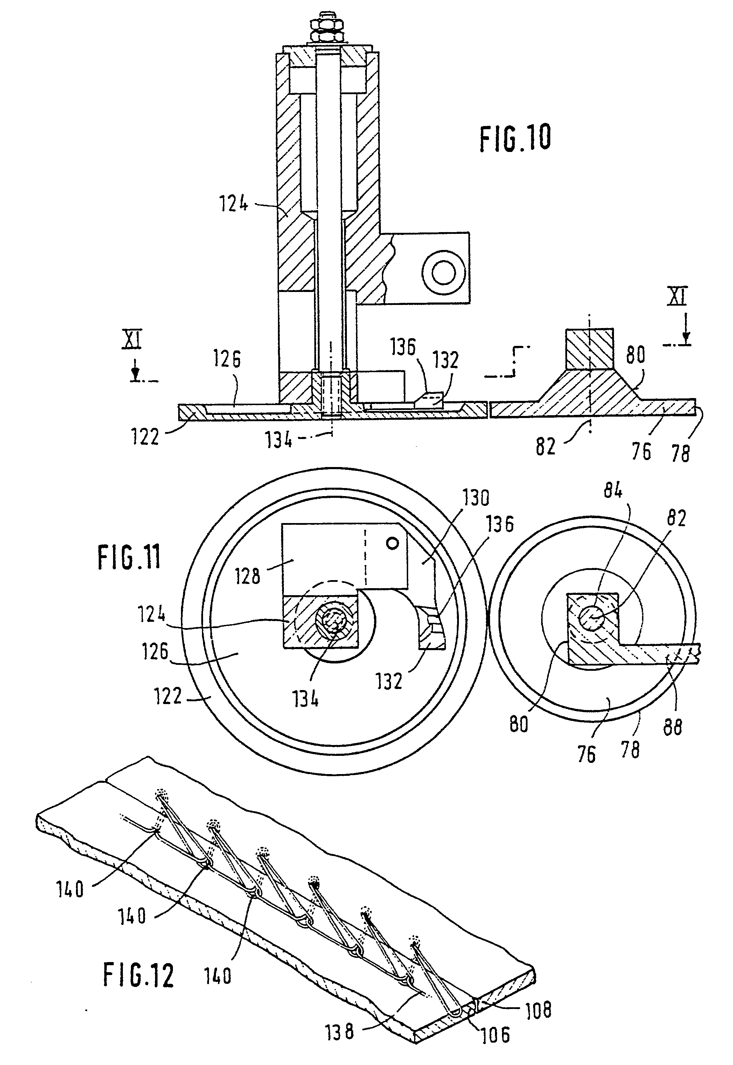

In den Fign. 8 und 9 ist eine Stützvorrichtung dargestellt, die den Gewebeüberstand beim Vernähen der Geweberänder gegen seitlichen Umkippen abstützt. Ferner ist in dieser Darstellung der feststehende Fuß mit der Platte (70), der als feststehendes Klemmteil fungiert, durch eine um die Rotationsachse (110) drehbare flache Scheibe (112) dargestellt. Der Aufbau der Scheibe (112) entspricht demjenigen der Scheibe (76), wobei der Durchmesser der Scheibe (112) größer als derjenige der Scheibe (76) ist. In diesem Fall sind beide Klemmteile drehbar, so daß die Umfangsflächen der beiden Scheiben (76, 112) jeweils auf einer Außenfläche der Geweberänder abrollen. Die Rotationswelle (114) der Scheibe (112) ist in einer Haltevorrichtung (116) gehalten, die am Kopfstück (14) des Gehäuses (10) der chirurgischen Nähmaschine befestigt ist. Die Stützvorrichtung für Gewebeüberstand besteht aus zwei Armen (118) und (120), von denen der Arm (118) am Teil (88) der Verstellvorrichtung (86) und der Arm (120) an der Haltevorrichtung (116) verschiebbar befestigt ist. Die Arme (118) und (120) sind in der Umfangszone der Scheiben (76) bzw. (112) abgewinkelt, ihre freien Enden folgen dem Scheibenkreisbogen ein Stück in Richtung der Klemmzone und erstrecken sich mit geringem Abstand oberhalb der betreffenden Scheibe. Die beiden Arme (118) und (120) sind in bezug auf die Scheibe (76) und (112) derart angeordnet, daß ihre dem Umfang der betreffenden Scheibe angepaßten freien Enden bei Draufsicht auf die Scheiben mit deren Rändern abschließen. Bei Bewegung der chirurgischen Nähmaschine in Richtung des Pfeiles A in Fig. 8 dreht sich die Scheibe (76) in Richtung B und die Scheibe (112) in Richtung C unter den unbeweglichen Armen (118) und (120) hinweg. Die über den Scheiben (76) und (112) überstehenden und zwischen ihnen eingeklemmten Geweberänder werden durch den Arm (118) beim Einstechen der Nadel (30) gegen seitliches Kippen in Richtung auf die Scheibe (76) abgestützt, während sie durch den Arm (120) gegen seitliches Kippen in Richtung auf die Scheibe (112) beim Herausbewegen der Nadel abgestützt werden. Durch die Stützvorrichtung wird erreicht, daß die Nadel (30) in gleicher Höhe durch die beiden hochstehenden Geweberänder (106) und (108) beim Vernähen derselben hindurchsticht und eine exakte Naht entsteht.

In Fig. 10 ist ein weiteres Beispiel für eine drehbare Scheibe (122) dargestellt, die anstelle des feststehenden Fußes bei der chirurgischen Nähmaschine als zweiter Klemmteil verwendet werden kann.

Die Scheibe (122) ist in Höhe der Scheibe (76) angeordnet und ihre Oberseite ist drehbar in einer Haltevorrichtung (124) befestigt. Die Scheibe (122) weist auf ihrer Oberseite eine ringförmige Vertiefung (126) auf. In der Vertiefung (126) befindet sich, ohne die Oberfläche der Scheibe (122) zu berühren, eine Platte (128), die fest an der Haltevorrichtung (124) befestigt ist. Bei Rotation der Scheibe (122) bewegt sich diese unter der Platte (128) hinweg. Die Platte (128) weist an ihrem der Scheibe (76) zugewandten Ende (130) eine nach oben weisende Erhebung (132) auf, in der sich eine radial zur Rotationsachse (134) der Scheibe (122) und radial zur Rotationsachse (82) der Scheibe (76) verlaufende Nut befindet, die als Nadelführung (136) fungiert und in der sich die Nadel (30) bei ihrer Oszillationsbewegung beegt. Die Haltevorrichtung (124) für die Scheibe (122) ist am Kopfstück (14) des Gehäuses (10) geeignet befestigt. Die beiden Scheiben sind derart aufeinander ausgerichtet, daß sich ihre Umfangsflächen in gleicher Ebene gegenüberliegen. Ihre Drehachsen liegen in einer gemeinsamen Ebene, die zur Nähmaschinenbewegung senkrecht gerichtet ist. Die Scheibe (122) ist eine freilaufende Scheibe, die beim Vernähen der Geweberänder auf der Außenfläche des Geweberandes (106) abrollt und sich daher dreht.

Nachfolgend soll kurz die Arbeitsweise der chirurgischen Nähmaschine erläutert werden.

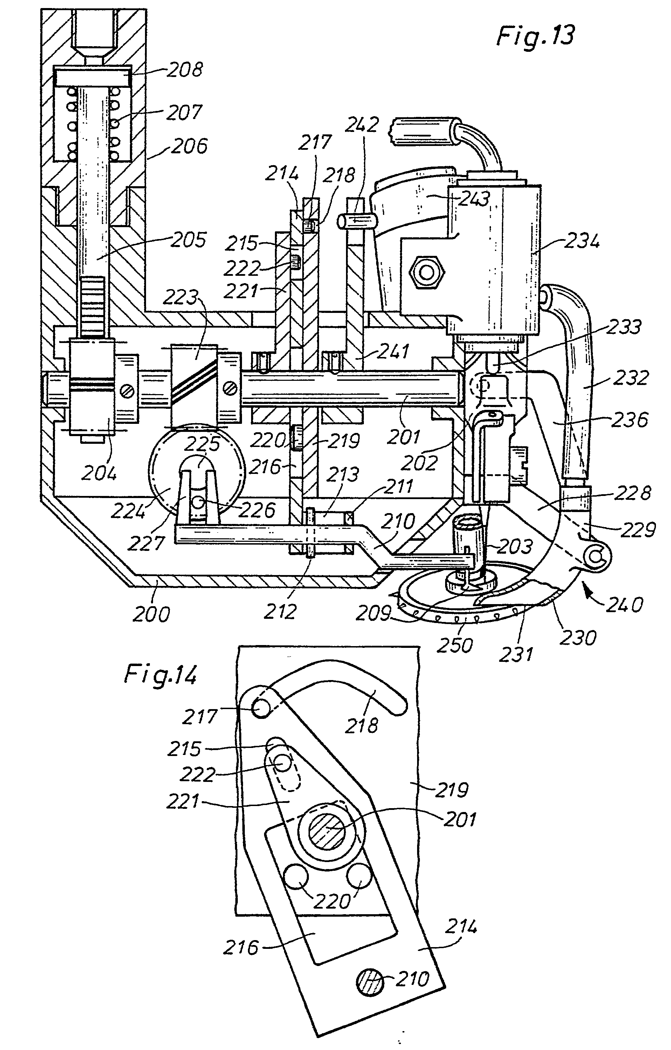

Mit Hilfe des Hebels (92) wird die Scheibe (76) von dem zweiten Klemmteil, das entweder der feststehende Fuß mit der Platte (70) oder ebenfalls eine drehbare Scheibe ist, abgerückt. Die zu vernähenden Geweberänder (106) und (108) werden jetzt hochstehend und aneinanderliegend zwischen die Scheibe (76) und die Platte (70) geführt, woraufhin mit Hilfe des Hebels (92) die Scheibe (76) in Richtung auf die Platte (70) bewegt wird. Die Andrückkraft der Scheibe (76) ist durch die Federkraft der Schraubenfeder (104) bestimmt. Jetzt werden die Bewegungsantriebe für die Nadel (30) und den Fadenfänger (18) in Gang gesetzt und die Geweberänder (106) und (108) bei Bewegung der chirurgischen Nähmaschine in Richtung des Pfeiles A in Fig. 2 miteinander vernäht. Zu Beginn eines Bewegungszyklus bewegt sich die Nadel (30) ausgehend von ihrer in Fig. 1 dargestellten Position in Richtung auf die hochstehenden Geweberänder (106) und (108), sticht durch diese hindurch und zieht den durch ihr Nadelöhr (32a) hindurchgeführten Nähfaden (34) durch die Geweberänder (106) und (108) hindurch. Zur Stabilisierung der Bewegung der Nadel (30) kann, wie in Fig. 11 dargestellt, dem Klemmteil (122) eine Nadelführung (136) zugeordnet sein. Wenn die Nadel (30) ihren Umkehrpunkt erreicht hat, bewegt sie sich durch die Geweberänder (106) und (108) hindurch zurück. Aufgrund der Reibung des Nähfadens (34) mit dem Gewebe wirft dieser sich auf und bildet eine Schlinge. In diese sich bei der Rückwärtsbewegung der Nadel (30) bildende Fadenschlinge greift der Fadenfänger (18) hinein, der sich während der Rückwärtsbewegung der Nadel (30) in Vorschubrichtung der chirurgischen Nähmaschine bewegt. Wenn die Nadel (30) die Geweberänder wieder verlassen hat, hat der Fadenfänger (18) die Fadenschlinge vollständig ergriffen. Jetzt beschreibt der Fadenfänger (18) eine Teilkreisbahn, so daß er die Schlinge über die Geweberänder (106) und (108) hinwegbewegt und auf die Einstichseite des Geweberandes (106) führt. Während dieser Zeit wird die chirurgische Nähmaschine in Richtung des Pfeiles A vorbewegt. Inzwischen hat die Nadel (30) ihren zweiten Umkehrpunkt erreicht und bewegt sich wieder auf die Geweberänder (106) und (108) zu. Zusammen mit dieser Bewegung der Nadel (30) bewegt sich der Fadenfänger (18) entgegengesetzt zur Vorschubrichtung A der chirurgischen Nähmaschine zurück, wobei die um die Zinken (40) und (44) gelegte Fadenschlinge durch den Haken (42) am Zinken (40) ein wenig mit zurückgezogen wird. Die Nadel (30) bewegt sich weiter auf die Geweberänder zu in den Zwischenraum zwischen die beiden Zinken (40) und (44) und damit durch die Fadenschlinge. Während die Nadel (30) in die Geweberänder (106) und (108) einsticht und durch diese hindurchdringt, beschreibt der Fadenfänger (18) eine Teilkreisbahn, die ihn über die Geweberänder (106) und (108) auf die andere Seite der Naht führt, wo er bei der nächsten Zurückbewegung der Nadel (30) den sich dabei aufwerfenden Faden wieder aufnehmen kann. Die miteinander vernähten hochstehenden Geweberänder (106) und (108) werden von dem Glattstreicher (22), der sich auf einer elliptischen Kreisbahn bewegt, flachgedrückt. Die so entstehende Naht ist in Fig. 12 dargestellt. Wie zu erkennen ist, ist die Schnittstelle zwischen den beiden Geweberändern (106) und (108) von Fadenschlingen überspannt. Die beiden Geweberänder (106) und (108) können sich somit nicht voneinander wegbewegen. An der Unterseite des Glattstreichers (22) können Düsen angeordnet sein, durch die Paste oder Kleber beim Flachdrücken der Geweberänder auf die Naht aufgetragen wird. Dies schützt die Naht vor Beschädigungen. Die in Fig. 12 dargestellte Naht hat den Vorteil, daß sie einfach gelöst werden kann. Wenn z. B. an dem Fadenende (138) gezogen wird, kann die gesamte Fadenmenge einfach aus den Geweberändern (106) und (108) herausgezogen werden. Das Fadenziehen ist bei dieser zuverlässig haltbaren Naht also sehr einfach.

Im Bereich der Platte (70) kann ein Fadenabschneider angeordnet sein, der nach Beendigung einer Naht die Fadenschlinge an einer Stelle abschneidet und dadurch die Voraussetzung für das Durchziehen des Fadens durch die Schlinge zum Verknoten der Naht schafft. Dies setzt jedoch voraus, daß die Nähmaschine über einen Motor verfügt, der mit einem Synchronisator verbunden ist und die Nähmaschine dann abschaltet, wenn der Fadenfänger (18) mitten über den Geweberändern steht.

Die Abstände der Einstiche (140) im Geweberand (106) werden von dem Vorschub der chirurgischen Nähmaschine in Richtung des Pfeiles A bestimmt. Dieser Vorschub kann entweder von Hand erfolgen, oder aber es ist auch möglich, daß die Scheibe (76) über einen Schrittschalter/Freilauf schrittweise angetrieben wird. Dieser Antrieb kann z. B. über die Bewegung des Glattstreichers (22) erfolgen. Durch die schrittweise Bewegung der Scheibe (76) in Richtung des Pfeils B in Fig. 7 wird die chirurgische Nähmaschine in Richtung des Pfeils A fortbewegt. Die Vorwärtsbewegung der chirurgischen Nähmaschine wird auch durch die Bewegung des Glattstreichers (22) unterstützt. Die an der Kopfseite 14 des Gehäuses (10) der chirurgischen Nähmaschine angeordneten beweglichen Teile, wie z.B. der Nadelhalter (16), der Fadenfänger (18), der zylindrische Körper (20), der Glattstreicher (22) sowie der Fuß mit der Platte (70) können mit Hilfe von Schnellverschlüssen schnell und einfach auf das Kopfstück (14) aufgesetzt werden. Dadurch ist eine einfache Sterilisation und ein schnelles Auswechseln der Teile möglich.

Ausführungsbeispiel 2

Das zweite Ausführungsbeispiel der chirurgischen Nähmaschine weist ein Gehäuse (200) auf, an dem ein nicht dargestellter Handgriff befestigt ist. Im Gehäuse ist eine Welle (201) gelagert, die an einem aus dem Gehäuse herausragenden Ende einen Nadelhalter (202) trägt. Am Nadelhalter (202) ist eine bogenförmige Nadel (203) angeklemmt, in die ein Nähfaden eingefädelt ist. Zum oszillierenden Antrieb der Welle (201) ist an ihrem dem Nadelhalter (202) abgekehrten Ende ein Ritzel (204) befestigt, das mit einer teilweise als Zahnstange ausgebildeten Kolbenstange (205) kämmt. Die Kolbenstange (205) ist Bestandteil eines am Gehäuse (200) befestigten einfachwirkenden Druckluftzylinders (206). Innerhalb des Druckluftzylinders (206) ist auf der Kolbenstange 205 eine Druckfeder (207) angeordnet, die sich am Kolben (208) abstützt.

Zur Bildung einer einfädigen Überdeck- bzw. Überwendlichnaht wirkt mit der Nadel (203) ein quer zur Nahtrichtung oszillierender und in Nahtrichtung hin- und herbewegter Fadenfänger (209) zusammen. Der Fadenfänger (209) ist an einer Schubstange (210) angeordnet, die in einer Buchse (211) axial verschiebbar gelagert ist. Die Schubstange (210) ist durch einen Querstift (212), der in einem Schlitz (213) der Buchse (211) geführt ist, gegen Verdrehen gesichert.

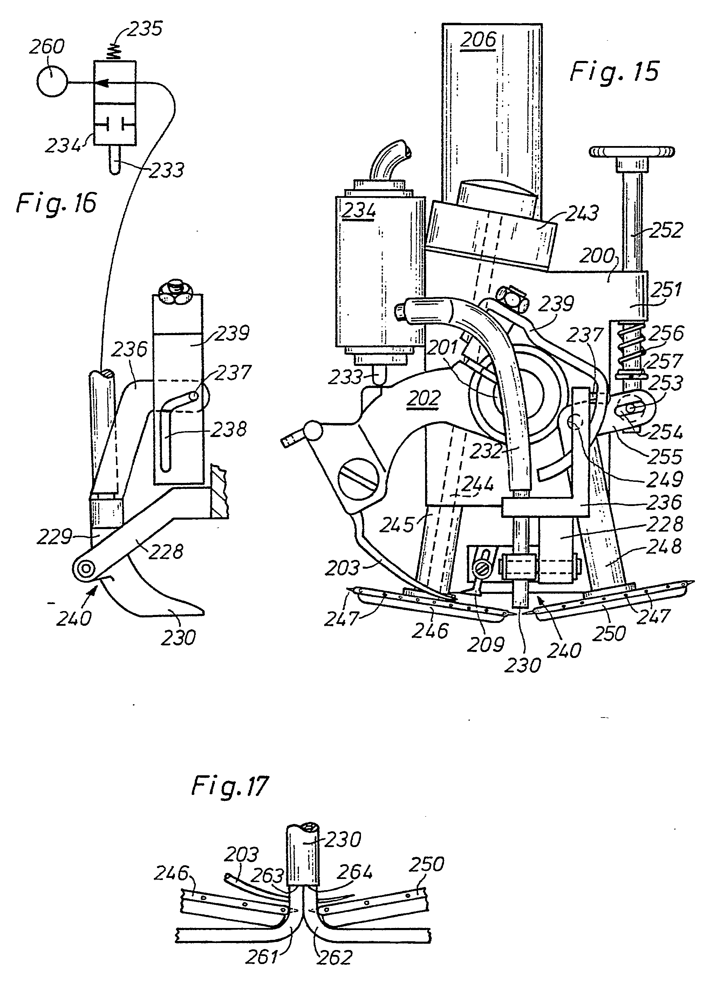

Die Buchse (211) ist an einer Schaukelplatte (214) befestigt, die gemäß Fig. 14 ein Langloch (215) und eine rechteckförmige Aussparung (216) enthält sowie einen quer abstehenden Stift (217) trägt. Der Stift (217) ist in einer bogenförmigen Nut (218) geführt, die in einer am Gehäuse (200) befestigten Führungsplatte (219) ausgebildet ist. Die Schaukelplatte (214) ist durch zwei in der Führungsplatte (219) befestigte, in die Aussparung (216) eingreifende Stifte (220) seitlich geführt. Auf der Welle (201) ist ein Antriebshebel (221) befestigt, der einen in das Langloch (215) eingreifenden Stift (222) trägt.

Auf der Welle (201) ist ein Schraubenrad (223) befestigt, das mit einem Schraubenrad (224) kämmt, welches auf einem quer zur Welle (201) angeordneten Bolzen (225) gelagert ist. An einer Stirnfläche des Schraubenrades (224) ist ein Stift (226) befestigt, der von einer auf der Schubstange (210) fest angeordneten Gabel (227) umgriffen ist.

An einem am Gehäuse (200) befestigten Tragarm (228) ist ein bogenförmiges Rohrstück (229) schwenkbar gelagert, dessen unteres Ende als ein keilförmiger, flach auslaufender Saugfuß (230) ausgebildet ist, der an der Unterseite eine Ansaugöffnung (231) aufweist. Auf das obere Ende des Rohrstückes (229) ist eine Schlauchleitung (232) aufgesteckt, die mit einem durch einen Stößel (233) steuerbaren 2/2-Wegeventil (234) verbunden ist. Das Wegeventil (234) wird gemäß Fig. 16 durch eine Feder (235) in Durchflußstellung gehalten, in der eine Saugluftquelle (260) mit dem Saugfuß (230) verbunden ist. Bei zurückgeschwenkter Nadel (203) ist der Stößel (233) durch den Nadelhalter (202) betätigt, wodurch das Wegeventil (234) in Sperrstellung geschaltet ist.

Am Rohrstück (229) greift ein L-förmiger Arm (236) an, der an seinem oberen Ende einen quer abstehenden Stift (237) trägt. Der Stift (237) ist in einer Kurvennut (238) einer am Nadelhalter (202) befestigten bogenförmigen Führungsplatte (239) geführt. Die Bauelemente (228 bis 239) bilden eine Saugluft-Haltevorrichtung (240).

Auf der Welle (201) ist ein Antriebshebel (241) befestigt, dessen oberes Ende gabelförmig ausgebildet ist. Der Antriebshebel (241) umgreift mit seinem gabelförmigen Ende einen quer abstehenden Mitnehmerhebel (242) eines bekannten und daher nicht näher erläuterten Schrittschaltwerkes (243). Das Schrittschaltwerk (243) treibt eine Welle (244) an, die in einer am Gehäuse (200) angeordneten Buchse (245) aufgenommen ist. Am unteren Ende der Welle (244) ist ein tellerförmiges Transportrad (246) befestigt, das an seiner Umfangsfläche eine Vielzahl kurzer Nadeln (247) aufweist.

Am Gehäuse (200) ist ein Träger (248) durch einen Gelenkbolzen (249) schwenkbar gelagert. Am unteren Ende des Trägers (248) ist ein gleichfalls tellerförmiges und mit Nadeln 247 versehenes Transportrad (250) frei drehbar gelagert. In einem Ansatz (251) des Gehäuses (200) ist eine axial bewegbare Betätigungsstange (252) angeordnet. Am unteren Ende der Betätigungsstange (252) ist ein quer abstehender Stift (253) angeordnet, der in ein Langloch (254) eingreift, welches in einem Arm (255) des Trägers (248) ausgebildet ist. Auf der Betätigungsstange (252) ist eine Druckfeder (256) angeordnet, die sich einerends am Ansatz (251) und anderenends an einem auf der Betätigungsstange (252) angeordneten Sicherungsring (257) abstützt und dadurch das Transportrad (250) in Richtung auf das Transportrad (246) drückt.

Das Transportrad (250) kann ohne weiteres ebenfalls mit dem Schrittschaltwerk (243) antriebsmäßig verbunden werden. Zu diesem Zweck müßte das Transportrad (250) auf einer im Träger (248) anzuordnenden Welle befestigt werden, die ihrerseits über ein Kardangelenk mit dem Schrittschaltwerk 243 verbunden wird.

Die Welle 201 kann auch auf eine andere Art und Weise als durch den Druckluftzylinder (206) oszillierend angetrieben werden, z. B. durch einen handbetätigten Schaltmechanismus oder einen Exzenterantrieb. Ferner läßt sich die oszillierende Antriebsbewegung durch eine von der Nähmaschine räumlich getrennte Antriebsvorrichtung erzeugen und durch eine biegsame Welle in die Nähmaschine einleiten.

Funktionsweise

Zum Einführen der miteinander zu verbindenden Geweberänder (261, 262) zwischen die beiden Transportträder (246, 250) wird durch Hochziehen der Betätigungsstange (252) das Transportrad (250) seitlich weggeschwenkt. Nach dem Einführen der Geweberänder (261, 262) wird die Betätigungsstange (252) losgelassen, wodurch die Druckfeder (256) das Transportrad (250) in Richtung des Transportrades (246) bewegt. Dabei dringen ein Teil der Nadeln (247) der beiden Transportträder (246, 250) mit ihren Spitzen in die Geweberänder (261, 262) ein, wodurch diese ohne nennenswert verletzt zu werden formschlüssig erfaßt werden.

In der in den Figuren 13 und 15 dargestellten Ruhestellung der Nähmaschine ist der Stößel (233) des Wegeventiles (234) betätigt, so daß sich das Wegeventil in Sperrstellung befindet. In dieser Schaltstellung des Wegeventils (234) herrscht an der Ansaugöffnung (231) des Saugfußes (230) der gleiche Luftdruck wie in der Umgebung, so daß der Saugfuß (230) hierbei keine Haltefunktion ausübt.

Zur Durchführung eines Stichbildevorganges wird die Nähmaschine durch kurzzeitiges Beaufschlagen des Druckluftzylinders (206) angetrieben, wodurch der Kolben (208) nach abwärts bewegt wird. Aus der unteren Stellung kehrt der Kolben (208) sodann durch die Wirkung der Druckfeder (207) wieder in seine Ausgangsstellung zurück. Infolge dieser Antriebsbewegung wird die Welle (201) um einen durch die Länge des Kolbenweges bestimmten Winkelbetrag oszillierend bewegt. Sobald der Nadelhalter (202) den Stößel (233) freigegeben hat, wird das Wegeventil (234) durch die Feder (235) in Durchflußstellung geschaltet, worauf an der Ansaugöffnung (231) des Saugfußes (230) Unterdruck herrscht. Aufgrund dieses Unterdruckes werden die Schnittkanten (263, 264) der Geweberänder (261, 262) vom Saugfuß (230) angesaugt und festgehalten.

Bevor die Nadel (203) in die Geweberänder einsticht, hat die sich gemeinsam mit der Nadel (203) bewegende Führungsplatte (239) aufgrund der Relativbewegung zwischen der Kurvennut (238) und dem Stift (237) den Arm (236) und damit den Saugfuß (230) nach aufwärts geschwenkt. Auf diese Weise hält der Saugfuß (230) die Geweberänder (261, 262) in der für eine einwandfreie Stichbildung erforderlichen Höhe bezüglich der Bewegungsbahn der Nadel (203).

Die Haltekraft des Saugfußes (230) ist so groß, daß die beim Einstechen der Nadel (203) sowie beim anschließenden Durchstechen und Zurückziehen auf die Geweberänder (261, 262) einwirkenden Kräfte vom Saugfuß (203) aufgefangen werden.

Während der Drehung der Welle (201) bewirkt der Antriebshebel (221) ein Verschwenken der Schaukelplatte (214) um die Stifte (220), wobei sie eine der Form der Nut (218) entsprechende Hubbewegung ausführt. Aufgrund dieser Bewegung der Schaukelplatte (214) führt der Fadenfänger (209) eine über die Schnittkanten (263, 264) und den flachen Teil des Saugfußes (230) hinweggehende bogenförmige Bewegung aus. Dieser Bewegung wird eine durch den oszillierenden Stift (226) hervorgerufene parallel zu den Schnittkanten (263, 264) verlaufende Bewegung überlagert, so daß der Fadenfänger (209) eine räumlich gekrümmte Bewegung ausführt.

Zu Beginn der Rückschwenkbewegung der Nadel (203) bildet der Nähfaden eine Schleife, in die der Fadenfänger (209) eindringt. Danach führt der Fadenfänger (209) die Schleife über den Saugfuß (230) hinweg auf die gegenüberliegende Seite und legt sie vor die wiedereinstechende Nadel (203). Bevor die Nadel (203) durch erneutes Beaufschlagen des Druckluftzylinders (206) eine neue Schwenkbewegung ausführt und dabei in die Fadenschleife eindringt, wird bei in Ausgangsstellung befindlicher Nadel (203) und somit bei in Sperrstellung befindlichem Wegeventil (234) über den Antriebshebel (241) das Schrittschaltwerk (243) betätigt, wodurch das Transportrad (246) um einen bestimmten Winkelbetrag gedreht wird. Aufgrund dieser Drehung des Transportrades (246) bewegt sich die Nähmaschine um das Maß der Stichlänge relativ zu den Geweberändern (261, 262). Durch diese Vorschubbewegung der Nähmaschine wird die über dem Saugfuß (230) liegende Fadenschleife vom Saugfuß abgezogen und so weit aufgeweitet, daß die Nadel (203) während des nächsten Stichbildevorganges sicher in die Fadenschleife einstechen kann.

**Patentansprüche**

1. Chirurgische Nähmaschine mit einem Gehäuse (10) sowie mit einer mit einem Nähfaden (34) verbundenen und oszillierend angetriebenen Nadel (30), die mit einem Fadenfänger (18) zusammenwirkt, und mit zwei Klemmteilen (20, 70), zwischen denen die Ränder (106, 108) des zu vernähenden Gewebes einklemmbar sind und deren Klemmzone quer zum Nahtverlauf von der Nadel durchquert wird, dadurch gekennzeichnet, daß die beiden Klemmteile (20, 70) am Gehäuse (10) befestigt sind und ihre dem zu vernähenden Gewebe zugewandten Stirnseiten freie Gleitflächen bilden, und daß wenigstens der eine Klemmteil ein drehbar befestigter zylindrischer Körper (20) ist, dessen Umfang (78) bei einer Bewegung der Nähmaschine in Nahtrichtung auf der Außenfläche des einen Geweberandes (108) abrollt.

2. Chirurgische Nähmaschine nach Anspruch 1, dadurch gekennzeichnet, daß die obere Stirnseite des zylindrischen Körpers (20) über eine Drehachse (84) mit einer Verstellvorrichtung (86) zum Ausrücken oder Einrücken des zylindrischen Körpers (20) in bezug auf den zweiten Klemmteil (70) hängend verbunden ist.

3. Chirurgische Nähmaschine nach Anspruch 2, dadurch gekennzeichnet, daß die Verstellvorrichtung (86) eine federelastische, einstellbare Einrichtung (98, 102, 104) zum Ändern der Klemmkraft des zylindrischen Körpers (20) aufweist.

4. Chirurgische Nähmaschine nach einem der Ansprüche 1 bis 3, dadurch gekennzeichnet, daß der zylindrische Körper (20) an der Einlaufseite des Gewebes in die Klemmzone abgesenkt ist und daß der Umfang des zylindrischen Körpers (78) als Mitnehmerring profiliert ist.

5. Chirurgische Nähmaschine nach einem der Ansprüche 1 bis 4, dadurch gekennzeichnet, daß an der oberen Stirnseite des zylindrischen Körpers (20) eine Nadelführungsfläche und/oder eine unbewegliche Nadelführung (136) angeordnet ist.

6. Chirurgische Nähmaschine nach einem der Ansprüche 1 bis 5, dadurch gekennzeichnet, daß an der oberen Stirnseite des zylindrischen Körpers (20) im Bereich der Klemmzone eine unbewegliche Stützvorrichtung (118) für Gewebeüberstand angeordnet ist.

7. Chirurgische Nähmaschine nach einem der Ansprüche 1 bis 6, dadurch gekennzeichnet, daß der zylindrische Körper (20) eine kreisförmige Scheibe (76) ist.

8. Chirurgische Nähmaschine nach einem der Ansprüche 1 bis 7, dadurch gekennzeichnet, daß an der Auslaufseite des Gewebes aus der Klemmzone ein Glattstreicher (22) für die Naht angeordnet ist.

9. Chirurgische Nähmaschine nach Anspruch 8, dadurch gekennzeichnet, daß der Glattstreicher (22) einen elliptisch bewegbaren Andrückschuh aufweist.

10. Chirurgische Nähmaschine nach Anspruch 9, dadurch gekennzeichnet, daß der zylindrische Körper (20) von dem Andrückschuh schrittweise antreibbar ist.

11. Chirurgische Nähmaschine nach einem der Ansprüche 1 bis 10, dadurch gekenzeichnet, daß der Fadenfänger (18) als gegabelter Arm ausgebildet ist, der sich axial und auf einer Teilkreisbahn bewegt und mit der Nadel (30) synchron so angetrieben ist, daß der Fadenfänger (18) den Nähfaden nach einer Durchdringung der Nadel durch das Gewebe von dieser aufnimmt, ihn über die Klemmzone legt und vor dem nächsten Durchdringen der Nadel (30) durch das Gewebe in Richtung der Einlaufseite des Gewebes in die Klemmzone vor die Nadel (13) legt.

12. Chirurgische Nähmaschine nach einem der Ansprüche 1 bis 11, dadurch gekennzeichnet, daß ein mit einem Griffstück (12) versehenes Gehäuse (10) vorgesehen ist, das Antriebs- und Übertragungselemente für den zylindrischen Körper (20), für einen Nadelhalter (16) und für den Fadenfänger (18) enthält und an dessen Kopfseite (14) der zylindrische Körper (20), der Nadelhalter (16) und der Fadenfänger (18) mittels Schnellverschlüssen jeweils lösbar angeordnet sind.

13. Chirurgische Nähmaschine nach einem oder mehreren der Ansprüche 1 bis 5, und 7 bis 10, dadurch gekennzeichnet, daß im Stichbildebereich der Nadel (203) wenigstens eine während eines jeden Stichbildevorganges wirksame Saugluft-Haltevorrichtung (240) für die Geweberänder vorgesehen ist.

14. Chirurgische Nähmaschine nach Anspruch 13, dadurch gekennzeichnet, daß die Haltevorrichtung (240) durch einen Antriebsmechanismus (236 bis 239) in vertikaler Ebene auf- und abbewegbar ist.

15. Chirurgische Nähmaschine nach Anspruch 14, dadurch gekennzeichnet, daß der Antriebsmechanismus (236 bis 239) der Haltevorrichtung (240) mit dem Triebwerk (201, 202) er Nähmaschine antriebsmäßig verbunden und die Hubhöhe der Haltevorrichtung (240) einstellbar ist.

16. Chirurgische Nähmaschine nach einem oder mehreren der Ansprüche 13 bis 15, dadurch gekennzeichnet, daß die Haltevorrichtung (240) ein im Takt der Nadelbewegung steuerbares Ventil (234) für die Zuleitung und Absperrung der Saugluft aufweist.

17. Chirurgische Nähmaschine nach einem oder mehreren der Ansprüche 13 bis 16, dadurch gekennzeichnet, daß die Haltevorrichtung (240) einen entgegen der Vorschubrichtung der Nähmaschine flach auslaufenden Saugfuß (230) mit einer an dessen Unterseite ausgebildeten Ansaugöffnung (231) aufweist.

## Claims

1. Surgical sewing machine including a housing (10) as well as a needle (30), which is linked to a thread and oscillatingly driven and co-acting with a thread pickup (18), and with two clamping members (20, 70) between which are clamped the edges (106, 108) of the fabric to be sewn and the clamping zone of which is crossed by the needle transversely to the course of the seam characterised in that the two clamping members (20, 70) are attached to the housing (10) and that their two sides facing towards the fabric to be sewn form free sliding surfaces, and that at least the one clamping member is a rotatably mounted cylindrical body (20) the circumference (78) of which rolls off on the outside surface of the one fabric edge (108) in the direction of the seam when the sewing machine is in motion.

2. Surgical sewing machine according to claim 1, characterised in that the upper end surface of the cylindrical body (20) is suspendedly connected via a rotary axis (84) to an adjusting device (86) for outward- or inward displacement of the cylindrical body (20) relative to the second clamping member (70).

3. Surgical sewing machine according to claim 2, characterised in that the adjusting device (86) includes a spring-elastic, adjustable device (98, 102, 104) to alter the clamping force of the cylindrical body (20).

4. Surgical sewing machine according to one of the claims 1 to 3, characterised in that the cylindrical body (20) is lowered at the running in side of the fabric into the clamping zone, and the circumference of the cylindrical body (78) is profiled to be a driver ring.

5. Surgical sewing machine according to one of the claims 1 to 4, characterised in that a thread-guiding surface and/or a fixed needle guide (136) is arranged at the upper end surface of the cylindrical body (20).

6. Surgical sewing machine according to one of the claims 1 to 5, characterised in that a fixed supporting means (118) for the fabric overlap is arranged at the upper end surface of the cylindrical body (20) in the region of the clamping zone.

7. Surgical sewing machine according to one of the claims 1 to 6, characterised in that the cylindrical body (20) is a circular disc (76).

8. Surgical sewing machine according to one of the claims 1 to 7, characterised in that a smoothing means (22) for the seam is arranged at the running-out side of the fabric of the clamping zone.

9. Surgical sewing machine according to claim 8, characterised in that the smoothing means (22) includes an elliptically displaceable pressure shoe.

10. Surgical sewing machine according to claim 9, characterised in that the cylindrical body (20) is driven step-by-step by the pressure shoe.

11. Surgical sewing machine according to one of the claims 1 to 10, characterised in that the thread pickup (18) is arranged to be a forked arm which moves axially and on a sectional circular path and which is driven synchronously with the needle (30) in such a manner that the thread pickup (18) picks up the thread from the needle after the latter has passed through the fabric, places it over the clamping zone and, prior to the next passing through the fabric by the needle (30) in the direction of the running-in side of the fabric, places it into the clamping zone in front of the needle (13).

12. Surgical sewing machine according to one of the claims 1 to 11, characterised in that a housing (10) including a handle (12) is provided, which accommodates the drive- and gearing elements for the cylindrical body (20), for the needleholder (16) and the thread pickup (18), and that at its head surface (14) are detachably arranged by means of quick-release fasteners the cylindrical body (20), the needleholder (16) and the thread pickup (18).

13. Surgical sewing machine according to one or several of the claims 1 to 5 and 7 to 10, characterised in that an vacuum-holding device (240) for the fabric edges is provided in the stitching region of the needle (203), which device is operative at least during each stitchforming process.

14. Surgical sewing machine according to claim 13, characterised in that the holding device (240) is movable upwards and downwards in a vertical plane by means of a drive mechanism (236 to 239).

15. Surgical sewing machine according to claim 14, characterised in that the drive mechanism (236 to 239) of the holding device (240) is connected to be driven by the drive (201, 202) of the sewing machine and that the lift height of the holding device (240) is adjustable.

16. Surgical sewing machine according to one or several of the claims 13 to 15, characterised in that the holding device (240) includes a valve (234), controlled in the cycle of the needle movement for supplying and shutting off the vacuum.

17. Surgical sewing machine according to one or several of the claims 13 to 16, characterised in that the holding device (240) has a suction foot (230), which runs flat against the forward thrust of the sewing machine and includes a suction aperture (231) formed at its bottom side.

**Revendications**

1. Machine à coudre chirurgicale comportant un boîtier (10) ainsi qu'une aiguille (30), reliée à un fil à coudre (34) et entraînée dans un mouvement oscillant, qui coopère avec un guide-fil (18) et comportant deux éléments de serrage (20, 70) entre lesquels sont serrés les bords (106, 108) du tissu à coudre et dont la zone de serrage est traversée par l'aiguille, perpendiculairement à la couture, caractérisée en ce que les deux éléments de serrage (20, 70) sont fixés sur le boîtier (10) et en ce que leurs faces frontales, tournées vers le tissu à coudre, forment des surfaces de glissement libres et en ce qu'au moins un élément de serrage est un corps cylindrique (20) fixé tournant, dont la périphérie (78) roule sur la surface extérieure d'un bord de tissu (108) lors d'un déplacement de la machine à coudre dans le sens de la couture.

2. Machine à coudre chirurgicale selon la revendication 1, caractérisée en ce que la face frontale supérieure du corps cylindrique (20), est reliée, en étant suspendue, par un axe de rotation (84), à un dispositif de réglage (86), pour le débrayage ou l'embrayage du corps cylindrique (20), par rapport au deuxième élément de serrage (70).

3. Machine à coudre chirurgicale selon la revendication 2, caractérisée en ce que le dispositif de réglage (86) comporte un dispositif réglable (98, 102, 104) élastique, pour modifier la force de serrage du corps cylindrique (20).

4. Machine à coudre chirurgicale selon l'une quelconque des revendications 1 à 3, caractérisée en ce que le corps cylindrique (20) est encastré dans la zone de serrage, sur le côté d'entrée du tissu et en ce que la périphérie du corps cylindrique (78) est profilée pour former un anneau d'entraînement.

5. Machine à coudre chirurgicale selon l'une quelconque des revendications 1 à 4, caractérisée en ce qu'une surface de guidage d'aiguille et/ou un organe de guidage d'aiguille (136), non mobile, est placé sur la face frontale supérieure du corps cylindrique (20).

6. Machine à coudre chirurgicale selon l'une quelconque des revendications 1 à 5, caractérisée en ce qu'un dispositif d'appui (118), non mobile, est placé sur la face frontale supérieure du corps cylindrique (20), dans la région de la zone de serrage, pour soutenir du tissu en surplomb.

7. Machine à coudre chirurgicale selon l'une quelconque des revendications 1 à 6, caractérisée en ce que le corps cylindrique (20) est un disque circulaire (76).

8. Machine à coudre chirurgicale selon l'une quelconque des revendications 1 à 7, caractérisée en ce qu'on place un lissoir (22) pour la couture, sur le côté où le tissu sort de la zone de serrage.

9. Machine à coudre chirurgicale selon la revendication 8, caractérisée en ce que le lissoir (22) présente un patin de pression se déplaçant en ellipse.

10. Machine à coudre chirurgicale selon la revendication 9, caractérisée en ce que le corps cylindrique (20) peut être entraîné pas-à-pas par le patin de pression.

11. Machine à coudre chirurgicale selon l'une quelconque des revendications 1 à 10, caractérisée en ce que le guide-fil (18) est un bras fourchu qui se déplace axialement sur une trajectoire en arc de cercle et est entraîné, de manière synchrone, avec l'aiguille (30), de manière que le guide-fil (18) saisisse le fil à coudre après que l'aiguille a traversé le tissu, l'applique sur la zone de serrage et le place devant l'aiguille (13), dans la zone de serrage, avant le prochain passage de l'aiguille (30) à travers le tissu, en direction du côté d'entrée du tissu.

12. Machine à coudre chirurgicale selon l'une quelconque des revendications 1 à 11, caractérisée en ce qu'on prévoit un boîtier (10), pourvu d'une poignée (12) et qui contient les éléments d'entraînement et de transmission pour le corps cylindrique (20), pour un porte-aiguille (16) et pour le guide-fil (18) et en ce que le corps cylindrique (20), le porte-aiguille (16) et le guide fil (18) sont placés, de manière amovible, par des raccords rapides, sur le côté de tête (14) du boîtier.

13. Machine à coudre chirurgicale selon l'une ou plusieurs des revendications 1 à 5, et 7 à 10, caractérisée en ce qu'on prévoit, dans la région de formation des points de l'aiguille (203), au moins un dispositif de maintien par aspiration d'air (240) pour les bords du tissu et qui entre en action à chaque formation d'un point.

14. Machine à coudre chirurgicale selon la revendication 13, caractérisée en ce que le dispositif de maintien (240) est déplaçable dans un mouvement de montée et de descente dans un plan vertical, par un mécanisme d'entraînement (236 à 239).

15. Machine à coudre chirurgicale selon la revendication 14, caractérisée en ce que le mécanisme d'entraînement (236 à 239) du dispositif de maintien (240) est relié en transmission, par le mécanisme d'entraînement (201, 202) de la machine à coudre et en ce que la hauteur de déplacement du dispositif de maintien (240) est réglable.

16. Machine à coudre chirurgicale selon l'une ou plusieurs des revendications 13 à 15, caractérisée en ce que le dispositif de maintien (240) comporte une vanne (234), commandée au rythme du déplacement de l'aiguille pour l'alimentation et la coupure de l'air d'aspiration.

17. Machine à coudre chirurgicale selon l'une ou plusieurs des revendications 13 à 16, caractérisée en ce que le dispositif de maintien (240) présente un pied d'aspiration (230) s'aplatissant dans le sens contraire au sens d'avance de la machine à coudre, avec une ouverture d'aspiration (231) formée sur sa face inférieure.

FIG.1

FIG.2

A →

EP 0 231 867 B1

FIG.3

FIG.4

FIG.5

FIG.6

FIG.7

FIG.8

FIG.9

FIG.10

124

126    136    132    80

XI

122    134    82    76    78

FIG.11

130    136    84    82

128

124
126
132

122    134    80    76    78    88

140

140

140

FIG.12    138    106    108

*Fig.13*

*Fig.14*

EP 0 231 867 B1

*Fig. 16*

*Fig. 15*

*Fig. 17*